# EUROPEAN PATENT SPECIFICATION

(11) **EP 1 163 916 B1**
(45) Date of publication and mention of the grant of the patent: **27.09.2006**
(21) Application number: 01113520.9
(22) Date of filing: 12.06.2001
(51) Int. Cl.: A61M 1/14, A61M 1/16

(54) **Apparatus for preparing solutions**
Vorrichtung zum Vorbereiten von Lösungen
Appareil pour préparer des solutions

(30) Priority: 15.06.2000 JP 2000179347
(43) Date of publication of application: 19.12.2001
(73) Proprietor: NIPRO CORPORATION, Kita-ku, Osaka-shi, Osaka-fu, 531-8510 (JP)
(72) Inventor: Sano, Yoshihiko, Osaka-shi, Osaka-fu 531-8510 (JP)
(74) Representative: Banzer, Hans-Jörg

(56) References cited:
- DE-C- 3 234 119
- US-A- 4 096 059
- PATENT ABSTRACTS OF JAPAN vol. 1996, no. 03, 29 March 1996 (1996-03-29) & JP 07 303694 A (NISSHO CORP), 21 November 1995 (1995-11-21)

## Description

### Technical Field of the Invention

The present invention relates to an apparatus and a method for preparing solutions by dissolving powder (including granulated powder) in a dissolving solution. The apparatus for preparing solutions in the present invention is especially suitable for preparing dialysate.

### Background of the Invention

Hitherto, preparation of solutions has been carried out in a tank system. In the tank system, prescribed quantities of a dissolving solution solvent and powder are introduced into a solution tank and stirred by a stirring pump or a stirring blade and mixed to form a solution. The prepared solution is transferred to a point of use by a delivery pump. At this time, the level of the surface of the solution in the solution tank is lowered, and a negative pressure is generated in the solution tank and thus outside air is introduced into the solution tank. The introduction of air occurs in the tank system because the solution tank is generally opened to the air to prevent breakage of the solution tank itself by a negative pressure generated therein. Therefore, in many cases, an air filter is provided at a portion opened to the air to prevent bacteria or the like contained in the outside air from entering. The use of an air filter results in problems and a high cost for replacing the air filter on a regular basis. As a matter of fact, there are cases where a filter that prevents only dust is used, or even no filter is used considering the problems and cost. In addition, in a tank system, when an attempt is made to prepare a large quantity of solution at a time, a large solution tank is necessary, thereby disadvantageously increasing the size of the apparatus itself. Moreover, since many stirring pumps and delivery pumps are necessary, the operating noise may disadvantageously be too loud.

From US-A-4,096,059 an apparatus for accurately proportioning and mixing fluids by using one or more double acting piston/cylinder units is known, whereby the cylinder of each double acting piston/cylinder unit is divided into chambers by the piston, and the volume of the cylinder swept by the piston at one end of the piston is lesser than that at the other end. Each double acting piston/cylinder unit defines a chamber which is divided by a movable partition into first and second compartments, the movable partition being the respective piston unit in combination with a rolling diaphragm. Alternatively, the movable partition may only be a diaphragm dividing the chamber into the two compartments. Furthermore, the apparatus comprises a plurality of lines or conduits connecting, on the one hand, both compartments of the chamber and, on the other hand, the two compartments of the chamber with inputs for a plurality of different fluids and with an output for outputting a mixture of the fluids, respectively.

With a view to the circumstances described above, an object of the present invention is to provide a cost effective apparatus and a corresponding method for preparing solutions in which replacement of an air filter for preventing bacteria or the like from entering into the solution tank is essentially unnecessary, and miniaturization of the entire system and lowering of operation noise are possible.

### Summary of the Invention

According to the present invention, this object is achieved by an apparatus for preparing solutions according to claim 1 and a method for preparing solutions according to claim 5. The dependent claims define preferred and advantageous embodiments of the present invention.

After dedicated studies, the inventor found that the above-described object can be achieved by utilizing a chamber which is divided by a movable partition into two compartments so that the solution can be prepared within a circuit containing these two compartments while substantially preventing outside air from entering therein, and reached the present invention. Stated differently, the present invention is an apparatus for preparing solutions comprising: a chamber which is divided by a movable partition into two compartments, a dissolving solution supply line for supplying a dissolving solution to the first compartment of the chamber, a solution preparing line connecting the first compartment and the second compartment of the chamber, a solution tank and a transporting pump provided in the solution preparing line, and a solution transporting line for transporting solution prepared in the solution tank and transported to the second chamber to the point of use.

The solution tank is provided, e.g. on its upper portion, with a powder supply means and preferably also with a liquid level detecting sensor therein. The dissolving solution supply line may be provided with a second dissolving solution supply line. The apparatus for preparing solutions of the present invention is preferably constructed in such a manner that the dissolving solution supply line, the solution preparing line, and the solution transporting line are further connected with a second chamber which is divided by a movable partition into two compartments so that the preparation of solution can be performed consecutively and continuously.

### Brief Description of the Drawings

Fig. 1 is a schematic system diagram showing an embodiment of the present invention.
Fig. 2 is a schematic system diagram showing another embodiment of the present invention.

### Detailed Description of the Invention

Referring now to the drawings, embodiments of the present invention will now be described.

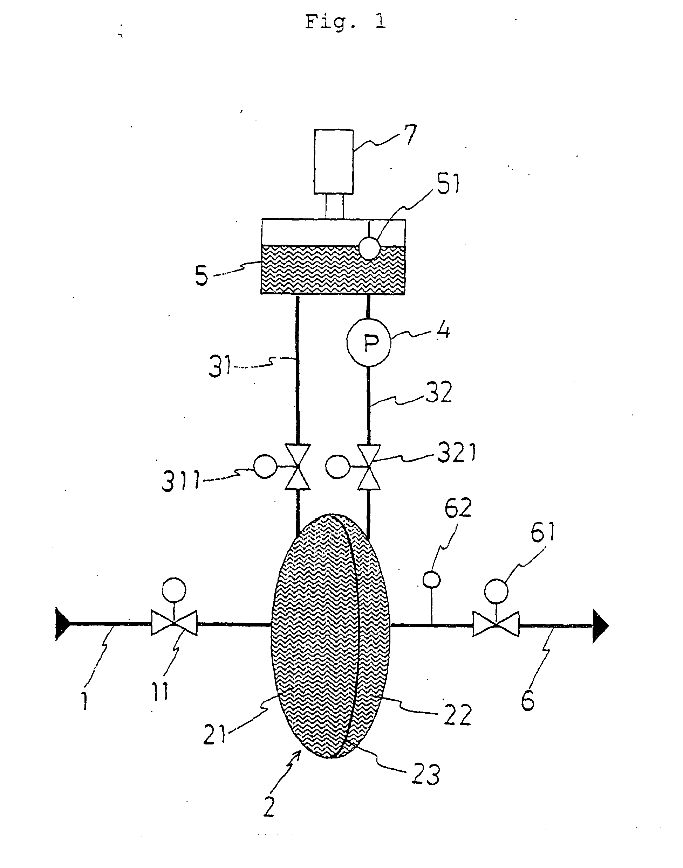

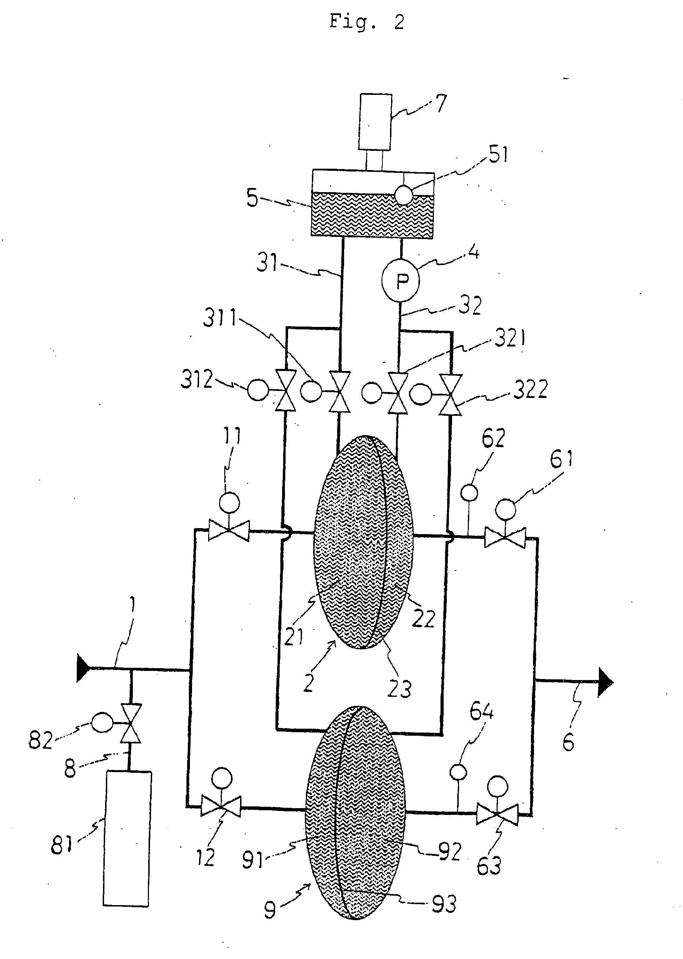

Fig. 1 is a circuit diagram showing an embodiment of the present invention, and Fig. 2 is a circuit diagram showing another embodiment of the present invention.

The apparatus for preparing solutions in the present invention comprises, as shown in Fig. 1, a chamber 2 the inside of which is divided by a movable partition 23 into two compartments 21, 22; a dissolving solution supply line 1 for supplying dissolving solution to the first compartment 21 of the chamber 2; a solution tank 5; a solution preparing line 31 for connecting the solution tank 5 and the first compartment 21 of the chamber 2, a solution preparing line 32 for connecting the solution tank 5 and the second compartment 22 of the chamber 2; a transporting pump 4 provided between the solution tank 5 and the second compartment 22; and a solution transporting line 6 for transporting a solution prepared in the solution tank 5 and transported to the second compartment 22 to the point of use. The transporting pump 4 may alternatively be provided between the first compartment 21 and the solution tank 5. In such a case, the action is slightly different.

The dissolving solution supply line 1, the solution preparing lines 31, 32, and the solution transporting line 6 are provided with switch valves 11, 311, 321, and 61, respectively. The solution tank 5 is preferably provided with a liquid level detecting sensor 51 therein, and with a powder supply means 7 on the upper portion thereof. The powder supply means 7 may be provided with an air filter (not shown) for preventing contamination caused by incoming outside air. For the operation of solution preparation, the switch valves 11, 311, and 61 are first opened and a dissolving solution, is supplied from a dissolving solution supply source (not shown) to the first compartment 21 of the chamber 2 via the dissolving solution supply line 1. Then, air contained in the second compartment 22 is discharged through the solution transporting line 6, and thus the movable partition 23 moves toward the second compartment 22. The movement of the movable partition 23 continues until the capacity of the second compartment 22 becomes zero. In other words, movement of the partition 23 continues until a quantity of dissolving solution equal to the capacity of the chamber 2 is filled in the first compartment 21. The supply of the dissolving solution is continued and the dissolving solution is then supplied to the solution tank 5 through the solution preparing line 31. When the level of the dissolving solution supplied to the solution tank 5 reaches a prescribed level that can be determined arbitrarily, the liquid level detecting sensor 51 is actuated to close the switch valves 11 and 61, and the switch valve 321 is opened and the transporting pump 4 is operated. The supply of powder to the solution tank 5 is continuously performed from the beginning to the end of the operation of the transporting pump 4, for example.

When the transporting pump 4 is operated, the solution in the solution tank 5 (mixed liquid of powder and dissolving solution) is supplied to the second compartment 22 through the solution preparing line 32, and simultaneously, dissolving solution in the first compartment 21 of the same quantity as the solution supplied to the second compartment 22 is supplied to the solution tank 5 through the solution preparing line 31. At this time, the movable partition 23 is caused to move toward the first compartment 21. The movement of the movable partition 23 continues until the capacity of the first compartment 21 becomes zero, i.e., until a quantity of the solution equal to the capacity of the chamber 2 is filled in the second compartment 22. During this solution preparing process, the liquid level in the solution tank 5 is maintained at a constant level, and thus an influx of outside air into the solution tank 5 hardly occurs.
The solution transporting line 6 between the second compartment 22 and the switch valve 61 is provided with a pressure gauge 62. When this pressure gauge 62 detects an increase of internal pressure of the second compartment 22 (when the capacity of the first compartment 21 becomes zero, the internal pressure of the second compartment 22 increases), the transporting pump stops, the switch valves 311 and 321 are closed, and the switch valves 11 and 61 are opened to again supply the dissolving solution to the first compartment 21 of the chamber 2 from the dissolving solution supply source through the dissolved liquid supply line 1. At this time, the movable partition 23 moves toward the second compartment 22, and the solution in the second compartment 22 is transported through the solution transporting line 6 to the point of use. The movement of the movable partition 23 and the transportation of the solution in the second compartment 22 to the point of use continue until the capacity of the second compartment 22 becomes zero, in other words, until a quantity of dissolving solution equal to the capacity of the chamber 2 is supplied to the first compartment 21. When the capacity of the second compartment 22 becomes zero, the internal pressure of the solution transporting line 6 suddenly drops. When the drop of the internal pressure in the solution transporting line 6 is detected by a pressure gauge 62, the switch valves 311 and 321 are opened, the switch valves 11 and 61 are closed and the transporting pump 4 is operated , and thus the dissolving solution is supplied through the solution preparing line 31 to the solution tank 5 and mixed with powder continuously supplied to the solution tank 5, and supplied to the second compartment 22 through the solution preparing line 32. The same procedures are repeated to prepare the solution.

The apparatus for preparing solution in the present invention may be constructed in such a manner that the dissolving solution supply line 1 is provided with a second dissolving solution supply line 8, as shown in Fig. 2. The second dissolving solution supply line 8 generally comprises a second dissolving solution supply source 81 and a switch valve 82. The apparatus for preparing solution in the present invention may be constructed in such a manner that a second chamber 9, the inside of which is divided by a movable partition 93 into two compartments, is connected to the dissolving solution supply line 1, the solution preparing lines 31, 32, and the solution transporting line 6 as shown in Fig. 2, so that the preparation of solution can be performed continuously. In the figure, the reference numerals 12, 312, 322 and 63 designate switch valves and 64 designates a pressure gauge.

In the case of the apparatus for preparing solutions shown in Fig. 2, preparation of a solution is continuously performed between the chamber 2 and the second chamber 9. First, the supply of the dissolving solution on the chamber 2 side of the apparatus is performed as in the case of the apparatus for preparing solutions shown in Fig. 1 with the switch valve 321 on the chamber 2 side of the apparatus and the switch valves 12, 312, 322, and 63 on the second chamber 9 of the apparatus closed. Then the switch valves 11 and 61 are closed and the switch valve 321 is opened and the transporting pump 4 caused to operate to prepare the solution. The supply of the dissolving solution to the first compartment 91 of the second chamber 9 is performed by opening the switch valves 12 and 63 while the switch valves 11 and 61 are closed and the transporting pump 4 is in operation. When a drop of the internal pressure in the solution transporting line 6 is detected by the pressure gauge 64, the supply of the dissolving solution to the first compartment 91 of the second chamber 9 terminates and the switch valves 12 and 63 are closed. When preparation of the solution on the chamber 2 side of the apparatus terminates, the transporting pump 4 stops, the switch valves 311 and 321 are closed, and the switch valves 11 and 61 are opened to supply the dissolving solution to the first compartment 21, and simultaneously, the solution is transported to the point of use. When a drop of the internal pressure of the solution transporting line 6 is detected by the pressure gauge 62, the supply of the dissolving solution to the first compartment 21 and transportation of the solution to the point of use terminate, and the switch valves 11 and 61 are closed. Simultaneously, the switch valves 312 and 322 on the second chamber 9 side of the apparatus are opened and the transporting pump 4 is operated to perform the preparation of the solution on the second chamber 9 side of the apparatus. When the preparation of solution on the second chamber 9 side of the apparatus terminates, the transporting pump 4 is stopped, the switch valves 312 and 322 are closed, and the switch valves 12 and 63 are opened to perform the supply of the dissolving solution to the first compartment 91, and simultaneously the solution is transported to the point of use. When a drop of the internal pressure of the solution transporting line 6 is detected by the pressure gauge 64, the supply of the dissolving solution to the first compartment 91 and the transportation of the solution to the point of use terminate, and the switch valves 12 and 63 are closed. Simultaneously, the switch valves 311 and 321 on the chamber 2 side of the apparatus are opened and the transporting pump 4 is operated to perform the preparation of the solution on the chamber 2 side of the apparatus. In the same manner, the supply of the dissolving solution on the chamber 2 side of the apparatus and the second chamber 2 side of the apparatus, the preparation of the solution and the transportation thereof to the point of use are alternatively repeated. The supply of the second dissolving solution can be per formed by opening the switch valve 82 as appropriate.

As is clear from the description above, the equipment for transporting liquid of the present invention is advantageous in terms of cost because an air filter and a stirring pump are not necessary, and the number of the delivery pumps can be reduced. Since a large solution tank is not necessary, miniaturization of the system itself is possible. Since there is only one delivery pump used, operating noise can significantly be reduced.

## Claims

1. An apparatus for preparing solutions comprising:
a chamber (2) which is divided by a movable partition (23) into first and second compartments (21, 22);
a first dissolving solution supply line (1) connected to the first comportment (21) of said chamber (2) for supplying a dissolving solution to the first compartment (21);
a solution preparing line (31, 32) connecting the first compartment (21) and the second compartment (22) of said chamber (2);
a solution tank (5) and a transporting pump (4) provided in said solution preparing line (31, 32);
a powder supply means (7) provided on the solution tank (5) for supplying powder to said solution tank (5); and
a solution transporting line (6) connected to the second compartment (22) of said chamber (2) for transporting a solution that has been prepared in said solution tank (5) by mixing the dissolving solution with the powder and that has been transported to the second compartment (22) from said second compartment (22) to a point of use.

2. An apparatus for preparing solutions claimed in claim 1, further comprising a liquid level detecting sensor (51) being provided in the solution tank (5).

3. An apparatus for preparing solutions claimed in anyone of claims 1-2, wherein the first dissolving solution supply line (1) is provided with a second dissolving solution supply line (8).

4. An apparatus for preparing solutions claimed in anyone of claims 1-3, further comprising a second chamber (9) which is divided by a movable partition (93) into two compartments (91, 92), said second chamber (9) being connected to the first dissolving solution supply line (1) , the solution preparing line (31, 32), and the solution transporting line (6) to enable the consecutive preparation of solution.

5. A method for preparing solutions using an apparatus according to any one of claims 1-4, comprising the steps:
supplying the dissolving solution from a dissolving solution supply source to the first compartment (21) of the chamber (2) via the first dissolving solution supply line (1);
discharging of air contained in the second compartment (22) of the chamber (2) via the solution transporting line (6), thereby moving the moveable partition (23) towards the second compartment (22) of the chamber (2) until a quantity of the dissolving solution substantially equal to a capacity of the chamber (2) is filled in the first compartment (21);
continuing the supply of the dissolving solution such that the dissolving solution is supplied to the solution tank (5) via the first compartment (21) of the chamber (2) and the solution preparing line (31);
supplying the powder to the solution tank (5) and mixing it with the dissolving solution in the solution tank (5) so as to prepare the solution;
operating the transporting pump (4) so as to supply the solution in the solution tank (5) to the second compartment (22) of the chamber (2) via the solution preparing line (32), and simultaneously supplying dissolving solution contained in the first compartment (21) of the chamber (2) of substantially the same quantity as the solution supplied to the second compartment (22) to the solution tank (5) via the solution preparing line (31) such that the moveable partition (23) of the chamber (2) is moved toward the first compartment (21) until a quantity of the solution substantially equal to the capacity of the chamber (2) is filled in the second compartment (22);
stopping the transporting pump (4); and
supplying again the dissolving solution from the dissolving solution supply source to the first compartment (21) of the chamber (2) via the dissolving solution supply line (1) such that the moveable partition (23) of the chamber (2) moves toward the second compartment (22), and the solution in the second compartment (22) of the chamber (2) is supplied through the solution transporting line (6) to the point of use.

## Patentansprüche

1. Vorrichtung zum Herstellen von Lösungen, umfassend:
eine Kammer (2), welche durch einen beweglichen Abschnitt (23) in eine erste und eine zweite Abteilung (21, 22) geteilt ist;
eine erste Zufuhrleitung (1) für die auflösende Lösung, welche mit der ersten Abteilung (21) der Kammer (2) verbunden ist, um der ersten Abteilung (21) eine auflösende Lösung zuzuführen;
eine Lösungsherstellungsleitung (31, 32), welche mit der ersten Abteilung (21) und der zweiten Abteilung (22) der Kammer (2) verbunden ist;
ein Lösungstank (5) und eine Transportpumpe (4), welche in der Lösungsherstellungsleitung (31, 32) vorhanden sind;
Pulverzufuhrmittel (7), welche auf dem Lösungstank (5) vorhanden sind, um dem Lösungstank (5) Pulver zuzuführen; und
eine Lösungstransportleitung (6), welche mit der zweiten Abteilung (22) der Kammer (2) verbunden ist, um eine Lösung, welche in dem Lösungstank (5) hergestellt worden ist, indem die auflösende Lösung mit dem Pulver gemischt worden ist, und welche zu der zweiten Abteilung (22) transportiert worden ist, von der zweiten Abteilung (22) zu einem Verwendungsort zu transportieren.

2. Vorrichtung zum Herstellen von Lösungen nach Anspruch 1, weiter einen ein Flüssigkeitsniveau erfassenden Sensor (51) umfassend, welcher in dem Lösungstank (5) vorhanden ist.

3. Vorrichtung zum Herstellen von Lösungen nach einem der Ansprüche 1-2, wobei die erste Zufuhrleitung (1) für die auflösende Lösung mit einer zweiten Zufuhrleitung (8) für die auflösende Lösung versehen ist.

4. Vorrichtung zum Herstellen von Lösungen nach einem der Ansprüche 1-3, weiter eine zweite Kammer (9) umfassend, welche durch einen beweglichen Abschnitt (93) in zwei Abteilungen (91, 92) geteilt ist, wobei die zweite Kammer (9) mit der ersten Zufuhrleitung (1) für die auflösende Lösung, der Lösungsherstellungsleitung (31, 32) und der Lösungstransportleitung (6) verbunden ist, um eine fortlaufende Herstellung der Lösung zu ermöglichen.

5. Verfahren zum Herstellen von Lösungen unter Verwendung einer Vorrichtung nach einem der Ansprüche 1-4, die Schritte umfassend:
Zuführen der auflösenden Lösung von einer Vorratsquelle der auflösenden Lösung zu der ersten Abteilung (21) der Kammer (2) über die erste Zufuhrleitung (1) für die auflösende Lösung;
Ablassen von Luft, welche in der zweiten Abteilung (22) der Kammer (2) enthalten ist, über die Lösungstransportleitung (6), wodurch der bewegbare Abschnitt (23) zu der zweiten Abteilung (22) der Kammer (2) bewegt wird, bis eine Menge der auflösenden Lösung, welche im Wesentlichen gleich einer Kapazität der Kammer (2) ist, in die erste Abteilung (21) gefüllt ist;
Fortführen des Zuführens der auflösenden Lösung, so dass die auflösende Lösung über die erste Abteilung (21) der Kammer (2) und die Lösungsherstellungsleitung (31) dem Lösungstank zugeführt wird;
Zuführen des Pulvers zu dem Lösungstank (5) und Mischen des Pulvers mit der auflösenden Lösung in dem Lösungstank (5), um so die Lösung herzustellen;
Betätigen der Transportpumpe (4), um so die Lösung in dem Lösungstank (5) der zweiten Abteilung (22) der Kammer (2) über die Lösungsherstellungsleitung (32) zuzuführen, und gleichzeitig Zuführen der auflösenden Lösung, welche in der ersten Abteilung (21) der Kammer (2) enthalten ist, im Wesentlichen in derselben Menge, wie die Lösung, welche der zweiten Abteilung (22) zugeführt ist, dem Lösungstank (5) über die Lösungsherstellungsleitung (31), so dass der bewegbare Abschnitt (23) der Kammer (2) zu der ersten Abteilung (21) bewegt wird, bis eine Menge der Lösung, welche im Wesentlichen gleich der Kapazität der Kammer (2) ist, in die zweite Abteilung (22) gefüllt ist;
Anhalten der Transportpumpe (4); und
wiederum Zuführen der auflösenden Lösung von der Vorratsquelle der auflösenden Lösung zu der ersten Abteilung (21) der Kammer (2) über die Zufuhrleitung (1) für die auflösende Lösung, so dass der bewegbare Abschnitt (23) der Kammer (2) zu der zweiten Abteilung (22) bewegt und die Lösung in der zweiten Abteilung (22) der Kammer (2) durch die Lösungstransportleitung (6) dem Verbindungsort zugeführt wird.

## Revendications

1. Appareil pour préparer des solutions comprenant :
une chambre (2) qui est divisée par une séparation mobile (23) en un premier et un second compartiments (21, 22) ;
une première conduite d'alimentation en solution de dissolution (1) raccordée au premier compartiment (21) de ladite chambre (2) afin d'alimenter le premier compartiment (21) en solution de dissolution ;
une conduite de préparation de solution (31, 32) raccordant le premier compartiment (21) et le second compartiment (22) de ladite chambre (2) ;
un réservoir de solution (5) et une pompe de transport (4) prévue dans ladite conduite de préparation de solution (31, 32) ;
un moyen d'alimentation en poudre (7) prévu sur le réservoir de solution (5) afin d'alimenter ledit réservoir de solution (5) en poudre ; et
une conduite de transport de solution (6) raccordée au second compartiment (22) de ladite chambre (2) afin de transporter une solution qui a été préparée dans ledit réservoir de solution (5) en mélangeant la solution de dissolution avec la poudre, et qui a été transportée jusqu'au second compartiment (22), à partir dudit second compartiment (22) jusqu'à un point d'utilisation.

2. Appareil pour préparer des solutions selon la revendication 1, comprenant en outre un capteur de détection de niveau de liquide (51) prévu dans le réservoir de solution (5).

3. Appareil pour préparer des solutions selon l'une quelconque des revendications 1 et 2, dans lequel la première conduite d'alimentation en solution de dissolution (1) est munie d'une seconde conduite d'alimentation en solution de dissolution (8).

4. Appareil pour préparer des solutions selon l'une quelconque des revendications 1 à 3, comprenant en outre une seconde chambre (9) qui est divisée par une séparation mobile (93) en deux compartiments (91, 92), ladite seconde chambre (9) étant raccordée à la première conduite d'alimentation en solution de dissolution (1), à la conduite de préparation de solution (31, 32), et à la conduite de transport de solution (6) afin de permettre la préparation consécutive de la solution.

5. Procédé pour préparer des solutions à l'aide d'un appareil selon l'une quelconque des revendications 1 à 4, comprenant les étapes consistant à :
fournir la solution de dissolution à partir d'une source d'alimentation en solution de dissolution au premier compartiment (21) de la chambre (2) par l'intermédiaire de la première conduite d'alimentation en solution de dissolution (1) ;
évacuer l'air contenu dans le second compartiment (22) de la chambre (2) par l'intermédiaire de la conduite de transport de solution (6), ce qui déplace la séparation mobile (23) vers le second compartiment (22) de la chambre (2) jusqu'à ce qu'une quantité de la solution de dissolution sensiblement égale à une capacité de la chambre (2) remplisse le premier compartiment (21) ;
poursuivre l'alimentation en solution de dissolution de telle sorte que la solution de dissolution soit fournie au réservoir de solution (5) par l'intermédiaire du premier compartiment (21) de la chambre (2) et de la conduite de préparation de solution (31) ;
alimenter le réservoir de solution (5) en poudre et la mélanger avec la solution de dissolution dans le réservoir de solution (5) de manière à préparer la solution ;
actionner la pompe de transport (4) de manière à fournir la solution qui se trouve dans le réservoir de solution (5) au second compartiment (22) de la chambre (2) par l'intermédiaire de la conduite de préparation de solution (32), et fournir simultanément la solution de dissolution contenue dans le premier compartiment (21) de la chambre (2) en quantité sensiblement égale à la solution fournie au second compartiment (22) au réservoir de solution (5) par l'intermédiaire de la conduite de préparation de solution (31) de telle sorte que la séparation mobile (23) de la chambre (2) soit déplacée vers le premier compartiment (21) jusqu'à ce qu'une quantité de solution sensiblement égale à la capacité de la chambre (2) remplisse le second compartiment (22) ;
arrêter la pompe de transport (4) ; et
fournir de nouveau la solution de dissolution provenant de la source d'alimentation en solution de dissolution au premier compartiment (21) de la chambre (2) par l'intermédiaire de la conduite d'alimentation en solution de dissolution (1) de telle sorte que la séparation mobile (23) de la chambre (2) se déplace vers le second compartiment (22), et que la solution qui se trouve dans le second compartiment (22) de la chambre (2) soit fournie par l'intermédiaire de la conduite de transport de solution (6) jusqu'au point d'utilisation.
